# EUROPEAN PATENT APPLICATION

(11) **EP 3 450 962 A1**
(43) Date of publication of application: **06.03.2019**
(21) Application number: 17789005.0
(22) Date of filing: 10.02.2017
(51) Int. Cl.: G01N 21/64, C12Q 1/04, G01N 15/14, G01N 21/78

(54) **FLUORESCENT PROBE FOR FLOW CYTOMETRY, AND METHOD FOR SCREENING FLUORESCENCE-LABELLED CELLS**

(30) Priority: 28.04.2016 JP 2016091356
(71) Applicant: National University Corporation Nagoya University, Nagoya-shi, Aichi 464-8601 (JP); Kurashiki Boseki Kabushiki Kaisha, Kurashiki-shi, Okayama 710-0054 (JP)
(72) Inventor: HAYASHI, Koichiro, Aichi 464-8601 (JP); SAKAMOTO, Wataru, Aichi 464-8601 (JP); YOGO, Toshinobu, Aichi 464-8601 (JP); MARUOKA, Hiroki, Osaka 572-0823 (JP)
(74) Representative: Dehns
(86) International application number: PCT/JP2017/004979
(87) International publication number: WO 2017/187716

(57) **Abstract**

The present invention relates to a fluorescent probe for flow cytometry that includes a carrier molecule and porphyrin bound to the carrier molecule and in which an excitation wavelength of the fluorescent probe for flow cytometry is in a range of 350 to 650 nm. The present invention also relates to a method for screening fluorescence-labeled cells using a flow cytometer that includes a step of fluorescently labeling cells with a fluorescent probe for flow cytometry and a step of screening fluorescence-labeled cells labeled with the fluorescent probe for flow cytometry using a flow cytometer, and in which the screening of the fluorescence-labeled cells using a flow cytometer is performed by irradiating the fluorescence-labeled cells with excitation light with a wavelength of 350 to 650 nm and detecting fluorescence.

## Description

### Technical Field

The present invention relates to a fluorescent probe for cytometry and a method for screening fluorescence-labeled cells.

### Background Art

In recent years, in vivo fluorescence imaging analysis using animals such as mice has been widely performed in the fields of cancer research, embryological research, and the like. The fluorescence imaging analysis is a major technique for analyzing information about positions at which cells such as stem cells including iPS cells, ES cells, and the like and disease-related cells including cancer cells, cirrhotic cells, and the like differentiate, grow, and metastasize after being transplanted into mice. Under current circumstances, only methods using the fluorescence imaging analysis can be used to observe the progression of transplanted cells, particularly with animals such as mice that are still alive, and such methods are regarded as particularly important analysis methods.

In general, fluorescence imaging analysis on transplanted cells in vivo is performed as follows: cells are labeled with a fluorescent probe in vitro in advance before the cells are transplanted, the cells are transplanted into a living organism such as a mouse, and the cells are observed using an in vivo fluorescence imaging apparatus. For example, Patent Document 1 discloses use of a porphyrin-containing complex obtained by binding anionic porphyrin, cationic organoalkoxysilane, and non-cationic silane together as a near-infrared fluorescent probe for observation of a living organism.

### Citation List

### Patent Document

Patent Document 1: JP 2013-136555A

### Disclosure of Invention

### Problem to be Solved by the Invention

However, under current circumstances, the fluorescence of the near-infrared fluorescent probe for observation of a living organism cannot be detected using an in vitro fluorescence imaging apparatus such as a fluorescence microscope or flow cytometer, and it is difficult to observe fluorescence-labeled cells bound to the near-infrared fluorescent probe in vitro.

In order to solve the foregoing conventional problems, the present invention provides a fluorescent probe for flow cytometry that can be used in flow cytometry analysis using a flow cytometer, and a method for screening fluorescence-labeled cells.

### Means for Solving Problem

The present invention relates to a fluorescent probe for flow cytometry including a carrier molecule, and porphyrin bound to the carrier molecule, wherein an excitation wavelength of the fluorescent probe for flow cytometry is in a range of 350 to 650 nm.

The present invention also relates to a method for screening fluorescence-labeled cells using a flow cytometer, and the method includes a step of fluorescently labeling cells with a fluorescent probe for flow cytometry, and a step of screening fluorescence-labeled cells labeled with the fluorescent probe for flow cytometry using a flow cytometer, wherein the fluorescent probe for flow cytometry includes a carrier molecule and porphyrin bound to the carrier molecule, an excitation wavelength of the fluorescent probe for flow cytometry is in a range of 350 to 650 nm, and the screening of the fluorescence-labeled cells using a flow cytometer is performed by irradiating the fluorescence-labeled cells with an excitation light with a wavelength of 350 to 650 nm and detecting fluorescence.

It is preferable that the carrier molecule is polysiloxane.

A configuration may be employed in which the fluorescent probe for flow cytometry is specifically bound to a surface of a cell so that the cell is labeled, or a configuration may be employed in which the fluorescent probe for flow cytometry is taken up by a cell so that the cell is labeled.

It is preferable that a fluorescence wavelength of the fluorescent probe for flow cytometry is in a range of 400 to 800 nm.

### Effects of the Invention

The present invention can provide a fluorescent probe for flow cytometry that can be used in flow cytometry analysis using a flow cytometer. Moreover, when cells are labeled with the fluorescent probe for flow cytometry, the fluorescence-labeled cells can be screened using a flow cytometer.

### Brief Description of Drawings

FIG. 1 shows FT-IR spectra of tetrakis(4-carboxyphenyl)porphyrin (TCPP) and a hybrid molecule (PPC HNPs) of TCPP and (3-mercaptopropyl)trimethoxysilane (MPTMS) measured using a Fourier transform infrared spectrophotometer (FT-IR).
FIG. 2 shows a ²⁹Si solid-state nuclear magnetic resonance (solid-state NMR) spectrum of PPS HNPs.
FIG. 3 shows a TG curve and a DTA curve of PPS HNPs.
FIG. 4A shows an absorption spectrum of a supernatant after FA-PEG/ICG-PPS HNPs production reaction, and FIG. 4B shows a calibration curve for a folic acid concentration.
FIG. 5 shows absorption spectra of PPS HNPs and a complex (FA-PEG/ICG-PPS HNPs) obtained by further binding indocyanine green (ICG) and folic acid (FA) to PPS HNPs.
FIG. 6A shows an excitation spectrum and a fluorescence spectrum of FA-PEG/ICG-PPS HNPs on a short wavelength side, and FIG. 6B shows an excitation spectrum and a fluorescence spectrum of FA-PEG/ICG-PPS HNPs on a long wavelength side.

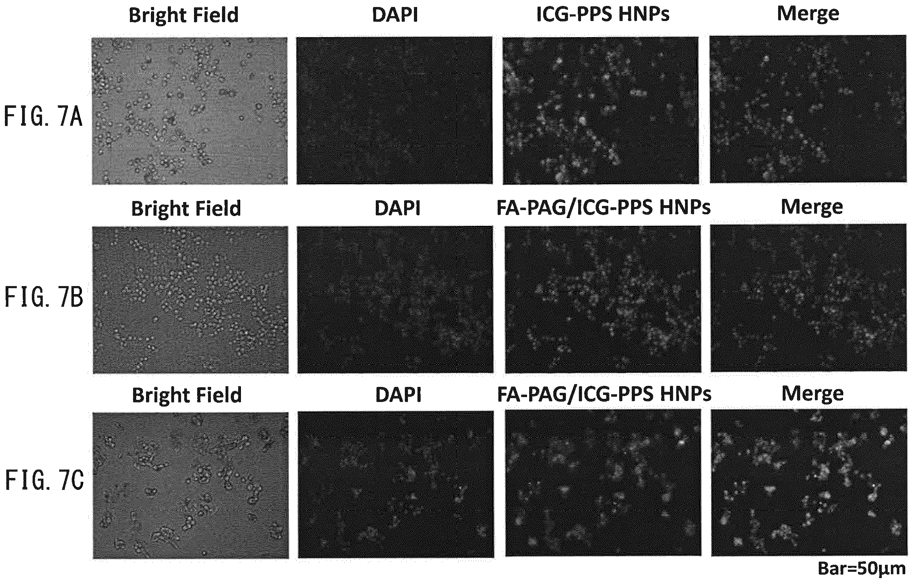

FIG. 7A shows a bright field image (Bright field) of RAW264.7 cells derived from a mouse macrophage, a fluorescence image (DAPI) of RAW264.7 cells stained with DAPI captured using a fluorescence microscope, a fluorescence image (ICG-PPS HNPs) of RAW264.7 cells labeled with a complex (ICG-PPS HNPs) obtained by further binding indocyanine green (ICG) to PPS HNPs captured using a fluorescence microscope, and an image (Merge) obtained by merging these three images together. FIG. 7B shows a bright field image (Bright field) of HeLa S3 cells derived from human cervical cancer, a fluorescence image (DAPI) of HeLa S3 cells stained with DAPI captured using a fluorescence microscope, a fluorescence image (FA-PEG/ICG-PPS HNPs) of HeLa S3 cells labeled with FA-PEG/ICG-PPS HNPs captured using a fluorescence microscope, and an image (Merge) obtained by merging these three images together. FIG. 7C shows a bright field image (Bright field) of HCT116 cells derived from human large intestine cancer, a fluorescence image (DAPI) of HCT116 cells stained with DAPI captured using a fluorescence microscope, a fluorescence image (FA-PEG/ICG-PPS HNPs) of HCT116 cells labeled with FA-PEG/ICG-PPS HNPs captured using a fluorescence microscope, and an image (Merge) obtained by merging these three images together.
[FIG. 8] FIG. 8A shows a result of analysis of RAW264.7 cells labeled with ICG-PPS HNPs using a flow cytometer (excitation wavelength: 405 nm; fluorescence wavelength: 670 nm). FIG. 8B shows a result of analysis of HeLa S3 cells labeled with FA-PEG/ICG-PPS HNPs using a flow cytometer (excitation wavelength: 405 nm; fluorescence wavelength: 670 nm). FIG. 8C shows a result of analysis of HCT116 cells labeled with FA-PEG/ICG-PPS HNPs using a flow cytometer (excitation wavelength: 405 nm; fluorescence wavelength: 670 nm).
[FIG. 9] FIG. 9A shows a result of analysis of RAW264.7 cells labeled with ICG-PPS HNPs using a flow cytometer (excitation wavelength: 405 nm; fluorescence wavelength: 710 nm). FIG. 9B shows a result of analysis of HeLa S3 cells labeled with FA-PEG/ICG-PPS HNPs using a flow cytometer (excitation wavelength: 405 nm; fluorescence wavelength: 710 nm). FIG. 9C shows a result of analysis of HCT116 cells labeled with FA-PEG/ICG-PPS HNPs using a flow cytometer (excitation wavelength: 405 nm; fluorescence wavelength: 710 nm).
[FIG. 10] FIG. 10A shows a result of analysis of RAW264.7 cells labeled with ICG-PPS HNPs using a flow cytometer (excitation wavelength: 405 nm; fluorescence wavelength: 780 nm). FIG. 10B shows a result of analysis of HeLa S3 cells labeled with FA-PEG/ICG-PPS HNPs using a flow cytometer (excitation wavelength: 405 nm; fluorescence wavelength: 780 nm). FIG. 10C shows a result of analysis of HCT116 cells labeled with FA-PEG/ICG-PPS HNPs using a flow cytometer (excitation wavelength: 405 nm; fluorescence wavelength: 780 nm).
[FIG. 11] FIG. 11A shows a result of analysis of RAW264.7 cells labeled with ICG-PPS HNPs using a flow cytometer (excitation wavelength: 640 nm; fluorescence wavelength: 780 nm). FIG. 11B shows a result of analysis of HeLa S3 cells labeled with FA-PEG/ICG-PPS HNPs using a flow cytometer (excitation wavelength: 640 nm; fluorescence wavelength: 780 nm). FIG. 11C shows a result of analysis of HCT116 cells labeled with FA-PEG/ICG-PPS HNPs using a flow cytometer (excitation wavelength: 640 nm; fluorescence wavelength: 780 nm).

### Description of the Invention

The inventors of the present invention found that a fluorescent probe that includes a carrier molecule and porphyrin bound to the carrier molecule and in which an excitation wavelength of the fluorescent probe for flow cytometry was in a range of 350 to 650 nm can be used to detect and screen fluorescence-labeled cells in fluorescence analysis using flow cytometry, and the present invention was thus achieved.

### Fluorescent probe for flow cytometry

A fluorescent probe for flow cytometry includes a carrier molecule and porphyrin bound to the carrier molecule.

The excitation wavelength of the fluorescent probe for flow cytometry is 350 to 650 nm, and preferably 380 to 580 nm from the viewpoint of suitability for flow cytometry analysis using a general-purpose flow cytometer. Moreover, the fluorescence wavelength of the fluorescent probe for flow cytometry is preferably in a range of 400 to 800 nm, and more preferably 450 to 680 nm, from the viewpoint of suitability for flow cytometry analysis using a general-purpose flow cytometer.

There is no particular limitation on the type of porphyrin, but porphyrin having a carboxyl group can be used from the viewpoint of suitability for fluorescence analysis using a flow cytometer. It should be noted that the term "porphyrin" is used to collectively refer to a ring compound in which four pyrrole rings are alternately bound to four methine groups at α positions, and derivatives thereof. The excitation wavelength of porphyrin having a carboxyl group is generally in a range of 400 to 650 nm, and its fluorescence wavelength is in a range of 600 to 740 nm.

For example, a compound represented by General Formula (I) below can be used as the porphyrin having a carboxyl group.

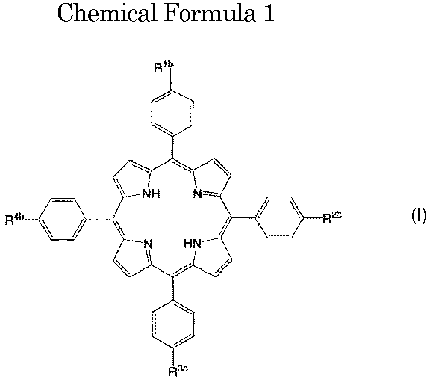

In General Formula (I) above, R^{1b}, R^{2b}, R^{3b}, and R^{4b} are optionally the same or different and represent a carboxyl group (COOH), a sulfo group (SO₃H), or a hydrogen atom (H) (it should be noted that a case where all of these are hydrogen atoms and a case where all of these are sulfo groups are excluded). It is preferable that, in General Formula (I) above, all of R^{1b}, R^{2b}, R^{3b}, and R^{4b} are carboxyl groups, or R^{1b} is a carboxyl group and R^{2b}, R^{3b}, and R^{4b} are hydrogen atoms. It is more preferable to use, as the porphyrin having a carboxyl group, a compound that is represented by General Formula (I) above and in which all of R^{1b}, R^{2b}, R^{3b}, and R^{4b} are carboxyl groups. The compound in which all of R^{1b}, R^{2b}, R^{3b}, and R^{4b} are carboxyl groups is called tetrakis(4-carboxyphenyl)porphyrin (TCPP).

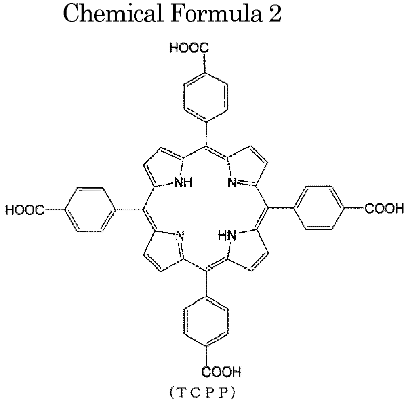

In addition, bilirubin, hemin, protoporphyrin, and the like can also be used as the porphyrin having a carboxyl group, for example.

The porphyrin having a carboxyl group used in the present invention is a known compound or can be easily manufactured using a known method. For example, commercially available compounds can be obtained from Tokyo Chemical Industry Co., Ltd., and the like.

The fluorescent probe of the present invention may also contain another fluorescent dye in addition to porphyrin. When another fluorescent dye is contained, fluorescence resonance energy transfer (FRET) should not occur between porphyrin and the other fluorescent dye. There is no particular limitation on the excitation wavelength of the other fluorescent dye (also referred to as "fluorescent dye b" hereinafter) as long as it is different from the excitation wavelength of porphyrin and FRET does not occur.

There is no limitation on the excitation wavelength of the fluorescent dye b as long as there is no influence on flow cytometry analysis using porphyrin. The excitation wavelength of the fluorescent dye b is preferably in a range of 600 to 850 nm, and more preferably in a range of 630 to 790 nm.

Fluorescent dyes for in vivo fluorescence imaging such as indocyanine compounds, coumarin, rhodamine, xanthene, hematoporphyrin, and fluorescamine may be used as the fluorescent dye b, for example.

It is preferable that the carrier molecule can bind to porphyrin and allows the original excitation wavelength of porphyrin to be maintained. Examples thereof include inorganic polymer such as polysilane, polygermane, polystannane, polysiloxane, polysilsesquioxane, polysilazane, polyborazirene, and polyphosphazene, and organic polymers such as polypyrrole, polyethylene glycol, and polysaccharides. For example, a silica polymer is formed through hydrolysis and polycondensation of silane as described later.

The fluorescent probe for flow cytometry may be specifically bound to the surface of a cell so that the cell is labeled. In this case, it is preferable that the fluorescent probe for flow cytometry includes a cell surface binding substance capable of binding to a substance for specific recognition of the cell surface. The fluorescent probe for flow cytometry specifically binds to the cell surface via a cell surface binding substance, and the cell can be thus labeled therewith. The "substance for specific recognition of the cell surface" refers to a protein, a lipid, a sugar chain, and/or a nucleic acid that is present on the surface of a specific cell. For example, a folic acid receptor, a transferrin receptor, an antigen, and the like that are specific to cancer cells are present on the surfaces of cancer cells. Cancer cells can be specifically labeled with a fluorescent probe including folic acid, transferrin, an antibody, or the like. A cell surface marker (membrane protein) and the like are present on the surfaces of stem cells such as iPS cells and ES cells. Stem cells such as iPS cells and ES cells can be specifically labeled with a fluorescent probe to which a molecule or the like that specifically binds to the cell surface marker of iPS cells or ES cells is bound.

The fluorescent probe for flow cytometry may be taken up by a cell so that the cell is labeled. Examples of such a cell include a macrophage, a dendritic cell, an immune cell, a cancer cell, and an iPS cell. Macropharges, dendritic cells, immune cells, cancer cells, iPS cells, or the like that have taken up the fluorescent probe can be used to confirm the dynamic behavior of macropharges, dendritic cells, or the like inside a body in an immune cell therapy.

The fluorescent probe for flow cytometry of the present invention can be produced by binding, preferably covalently binding, a carrier molecule to porphyrin to form a complex of the carrier molecule and porphyrin. A cell surface binding substance may be bound to the carrier molecule in the complex of the carrier molecule and porphyrin as needed. It is preferable that the excitation wavelength and the fluorescence wavelength of porphyrin in the fluorescent probe for flow cytometry of the present invention barely change before and after the carrier molecule is bound to porphyrin.

When the carrier molecule is polysiloxane, the fluorescent probe for flow cytometry of the present invention can be produced as described below, for example.

First, silane having an amino group and porphyrin having a carboxyl group are reacted to obtain silane including porphyrin in its molecule (also referred to as "porphyrin-silane" hereinafter). Specifically, silane having an amino group and porphyrin having a carboxyl group are dissolved in a solvent, and a condensing agent is added thereto to initiate an amidation reaction. An example of the solvent is N,N-dimethylformamide (DMF). An example of the condensing agent is carbodiimide. An example of carbodiimide is N,N'-dipropylcarbodiimide, but there is no particular limitation thereto. Succinimide or the like may be added in order to reduce by-products. An example of succinimide is N-hydroxysuccinimide, but there is no particular limitation thereto. The reaction temperature is preferably 20 to 150°C, and more preferably 20 to 80°C, for example, from the viewpoint of synthesis cost, but there is no particular limitation thereto. The reaction time is preferably 1 to 24 hours, and more preferably 3 to 15 hours, for example, but there is no particular limitation thereto. After the reaction, the product is collected as a precipitate through centrifugation, and porphyrin-silane thus can be obtained.

In the above-mentioned reaction, the molar ratio of the silane having an amino group to the porphyrin having a carboxyl group (silane having an amino group:porphyrin having a carboxyl group) is preferably 4:1 to 1:1, more preferably 4:1 to 2:1, and even more preferably 4:1.

Next, a complex of polysiloxane and porphyrin is obtained through hydrolysis and polycondensation reaction between the porphyrin-silane obtained as described above and silane having one or more functional groups (also referred to as "functional silane" hereinafter). Specifically, the porphyrin-silane and the functional silane are dissolved in a solvent, and then an alkali solution is added thereto to initiate a reaction. An example of the solvent is DMF. Examples of the alkali solution include an aqueous solution of ammonia and an aqueous solution of sodium hydroxide whose pH is 8 or higher. The reaction temperature is preferably 20 to 200°C, and more preferably 60 to 80°C, for example, from the viewpoint of synthesis cost, but there is no particular limitation thereto. The reaction time is preferably 1 to 72 hours, and more preferably 3 to 24 hours, for example, but there is no particular limitation thereto. After the reaction, the product is collected as a precipitate through centrifugation, and the complex of polysiloxane and porphyrin in which porphyrin is covalently bound to polysiloxane thus can be obtained.

In the above-mentioned reaction, the molar ratio of the porphyrin-silane to the functional silane (porphyrin-silane:functional silane) is preferably 1:2 to 1:100, more preferably 1:21 to 1:50, and even more preferably 1:30 to 1:40.

There is no particular limitation on the silane having an amino group as long as an amino group is included. For example, a compound represented by General Formula (II) below can be favorably used.

Xᵢ-Si(R^{2a})ⱼ(OR^{1a})₍₄₋ᵢ₋ⱼ₎ (II)

In General Formula (II), X represents a group represented by H₂NCₘH2ₘ-, H₂NCₙH₂ₙ-HNCₘH₂ₘ-, or Ph-NHCₘH₂ₘ- (where Ph represents a phenyl group). In particular, a group represented by H₂NCₘH₂ₘ- or H₂NCₙH₂ₙ-HNCₘH2ₘ- is favorable. m and n are the same or different and represent an integer of 1 to 6. m is preferably 1, 2, 3, or 4, and more preferably 1, 2, or 3. n is preferably 1, 2, 3, or 4, and more preferably 1, 2, or 3. H₂NCₘH2ₘ-, H₂NCₙH2ₙ-HNCₘH₂ₘ-, and Ph-NHCₘH₂ₘ- are preferably H2N(CH2)ₘ-, H₂N(CH₂)ₙ-HN(CH₂)ₘ-, and Ph-NH(CH₂)ₘ-, respectively.

R^{1a} and R^{2a} are the same or different and represent an alkyl group having 1 to 6 carbon atoms. The alkyl group may be a linear chain or a branched chain, and is preferably a linear chain. It is preferable that the alkyl group has 1, 2, 3, or 4 carbon atoms. Specific examples of the alkyl group having 1 to 6 carbon atoms include a methyl group, an ethyl group, an n-propyl group, an isopropyl group, an n-butyl group, an isobutyl group, a tert-butyl group, a sec-butyl group, an n-pentyl group, a 1-ethylpropyl group, an isopentyl group, a neopentyl group, an n-hexyl group, a 1,2,2-trimethylpropyl group, a 3,3-dimethylbutyl group, a 2-ethylbutyl group, an isohexyl group, and a 3-methylpentyl group. In particular, a methyl group, an ethyl group, an n-propyl group, an isopropyl group, an n-butyl group, and an isobutyl group are preferable.

i represents 1 or 2, and j represents 0 or 1 (it should be noted that a relationship (4-i-j)≥2 is satisfied). That is, (i,j) represents (1,0), (1,1) or (2,0).

Examples of the compound represented by General Formula (II) include 3-aminopropyltriethoxysilane (APTES), 3-(2-aminoethylamino)propyldimethoxymethylsilane, 3-(2-aminoethylamino)propyltriethoxysilane, 3-(2-aminoethylamino)propyltrimethoxysilane, 3-aminopropyldiethoxymethylsilane, 3-aminopropyltrimethoxysilane, and trimethoxy[3-(phenylamino)propyl]silane. In particular, 3-aminopropyltriethoxysilane (APTES) and/or 3-aminopropyltrimethoxysilane are preferable.

For example, commercially available compounds manufactured by Tokyo Chemical Industry Co., Ltd. can be used as the above-described silane having an amino group. The silanes having an amino group can be used alone or in combination of two or more.

There is no particular limitation on the functional silane as long as one or more functional groups are included. The functional silane may be monofunctional silane having one functional group or polyfunctional silane having two or more functional groups. A compound represented by General Formula (III):

Yₚ-Si(R^{2c})_{q}(OR^{1c})_{(4-p-q)} (III)

can be favorably used as the silane having a functional group, for example.

In General Formula (III) above, Y represents a group represented by CH₂=CH-, a group represented by CH₂=CHCH₂-, a group including alkene, a group including thiol, a group including disulfide, a group including amine, a group including ester, a group including amide, a group including carboxylic acid, a group including urea, a group including thiourea, a group represented by OCNCH₂CH₂-, a group represented by ClC_{α}H_{2α}-, a group represented by HSC_{β}H_{2β}-, a group represented by CF₃C_{γ}F_{2γ}-C_{δ}H_{2δ}-, a group represented by CH₂=C(CH₃)COOC_{ε}H_{2ε}-, a group represented by CH₂=CHCOOC_{ζ}H_{2ζ}-, a group represented by HN-CONH-C_{η}H_{2η}-, a group represented by Chemical Formula (a) below, a group represented by Chemical Formula (b) below, an alkyl group having 1 to 18 carbon atoms, or a phenyl group.

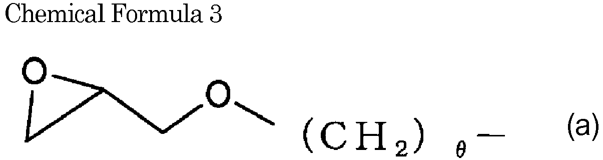

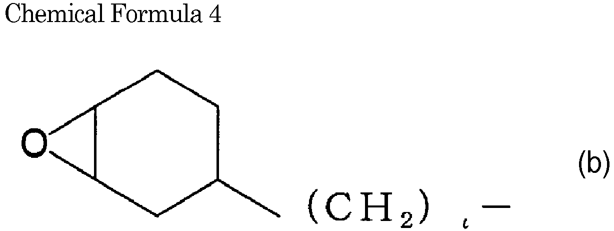

In the description above, α, β, δ, ε, ζ, η, θ, and 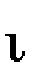 independently represent an integer of 1 to 6, and preferably an integer of 1, 2, 3, or 4. γ represents an integer of 0 to 8, and preferably an integer of 0, 1, 2, 3, 4, 5, 6, or 7. The alkyl group having 1 to 18 carbon atoms preferably has 1 to 12 carbon atoms, and more preferably 1, 2, 3, 4, 5, 6, 7, or 8 carbon atoms. The alkyl group having 1 to 18 carbon atoms may be a linear chain or a branched chain, and is preferably a linear chain. ClC_{α}H_{2α}-, HSC_{β}H_{2β}-, CF₃C_{γ}F_{2γ}-C_{δ}H_{2δ}-, CH₂=C(CH₃)COOCH_{2ε}-, CH₂=CHCOOC_{ζ}H_{2ζ}-, and HN-CONH-C_{η}H_{2η}- above are preferably Cl(CH₂)_{α}-, HS(CH₂)_{β}-, CF₃(CF₂)_{γ}-(CH₂)_{δ}-, CH₂=C(CH₃)COO(CH₂)_{ε}-, CH₂=CHCOO(CH₂)_{ζ}-, and HN-CONH-(CH₂)_{η}-, respectively.

R^{1c} represents an alkyl group having 1 to 6 carbon atoms or -(CH₂)_{τ}-OCH₃, and R^{2c} represents an alkyl group having 1 to 6 carbon atoms or a phenyl group. R^{1c} and R^{2c} may be the same or different. In both R^{1c} and R^{2c}, the alkyl group having 1 to 6 carbon atoms may be a linear chain or a branched chain, and is preferably a linear chain. An alkyl group having 1, 2, 3, or 4 carbon atoms is preferable. Specific examples of the alkyl group having 1 to 6 carbon atoms include a methyl group, an ethyl group, an n-propyl group, an isopropyl group, an n-butyl group, an isobutyl group, a tert-butyl group, a sec-butyl group, an n-pentyl group, a 1-ethylpropyl group, an isopentyl group, a neopentyl group, an n-hexyl group, a 1,2,2-trimethylpropyl group, a 3,3-dimethylbutyl group, a 2-ethylbutyl group, an isohexyl group, and a 3-methylpentyl group. In particular, a methyl group, an ethyl group, an n-propyl group, an isopropyl group, an n-butyl group, and an isobutyl group are preferable. τ represents an integer of 1 to 4 (preferably 1, 2, or 3).

p represents 1, 2, or 3, and q represents 0, 1, or 2 (it should be noted that a relationship (4-p-q)≥1 is satisfied). That is, (p,q) represents (1,0), (1,1), (1,2), (2,0), (2,1) or (3,0).

A compound represented by General Formula (IV) can also be favorably used as the functional silane, for example.

Z-SiCl₃ (IV)

In General Formula (IV) above, Z represents a group represented by CH₂=CH-, a group represented by CH₂=CHCH₂-, a group including alkene, a group including thiol, a group including disulfide, a group including amine, a group including ester, a group including amide, a group including carboxylic acid, a group including urea, a group including thiourea, a group represented by ClC_{κ}H_{2κ}-, a group represented by CF₃C_{λ}F_{2λ}-Q_{ξ}H_{2ξ}-, an alkyl group having 1 to 18 carbon atoms, a phenyl group, or a cyclohexyl group. κ and ξ independently represent an integer of 1 to 6, and preferably an integer of 1, 2, 3, or 4. λ represents an integer of 0 to 8, and preferably an integer of 0, 1, 2, 3, 4, 5, 6, or 7. The alkyl group having 1 to 18 carbon atoms preferably has 1 to 12 carbon atoms, and more preferably 1, 2, 3, 4, 5, 6, 7, or 8 carbon atoms. The alkyl group having 1 to 18 carbon atoms may be a linear chain or a branched chain, and is preferably a linear chain. ClC_{κ}H_{2κ}- and CF₃C_{λ}F_{2λ}-C_{ξ}H_{2ξ}- above are preferably Cl(CH₂)_{κ}- and CF3 (CF₂)_{λ}(CH₂)_{ξ}-, respectively.

An N-[2-(N-vinylbenzylamino)ethyl]-3-aminopropyltrimethoxysilane hydrochloride may also be used as the polyfunctional silane.

Specific examples of the compound represented by General Formula (III) include tetramethoxysilane, tetraethoxysilane, tetrapropoxysilane, tetrabutoxysilane, tetraisopropoxysilane, allyltriethoxysilane, allyltrimethoxysilane, diethoxymethylvinylsilane, dimethoxymethylvinylsilane, triethoxyvinylsilane, vinyltrimethoxysilane, vinyltris(2-methoxyethoxy)silane, (chloromethyl)triethoxysilane, 3-chloropropyldimethoxymethylsilane, 3-chloropropyltriethoxysilane, 2-(3,4-epoxycyclohexyl)ethyltrimethoxysilane, 3-mercaptopropyl(dimethoxy)methylsilane, (3-mercaptopropyl)triethoxysilane, (3-mercaptopropyl)trimethoxysilane, 3-(triethoxysilyl)propyl isocyanate, 3-(trimethoxysilyl)propyl methacrylate, triethoxy-1H,1H,2H,2H-tridecafluoro-n-octylsilane, 3-(trimethoxysilyl)propyl acrylate, 3-trimethoxysilylpropylchloride, 1-[3-(trimethoxysilyl)propyl] urea, trimethoxy(3,3,3-trifluoropropyl)silane, 3-ureidepropyltriethoxysilane, diethoxy(3-glycidyloxypropyl)methylsilane, 3-glycidyloxypropyl(dimethoxy)methylsilane, 3-glycidyloxypropyltrimethoxysilane, 2-cyanoethyltriethoxysilane, diacetoxydimethylsilane, diethoxydimethylsilane, dimethoxydimethylsilane, dimethoxydiphenylsilane, dimethoxymethylphenylsilane, dodecyltriethoxysilane, hexyltrimethoxysilane, octadecyltriethoxysilane, octadecyltrimethoxysilane, n-octyltriethoxysilane, pentyltriethoxysilane, triacetoxymethylsilane, triethoxyethylsilane, trimethoxy(methyl)silane, trimethoxyphenylsilane, and trimethoxy(propyl)silane. In particular, 3-mercaptopropyl(dimethoxy)methylsilane, (3-mercaptopropyl)triethoxysilane, (3-mercaptopropyl)trimethoxysilane, and the like are preferable.

Specific examples of the compound represented by General Formula (IV) include allyltrichlorosilane, trichlorovinylsilane, 3-chloropropyltrichlorosilane, trichloro(1H,1H,2H,2H-heptadecafluorodecyl)silane, trichloro(1H,1H,2H,2H-tridecafluoro-n-octyl)silane, butyltrichlorosilane, cyclohexyltrichlorosilane, decyltrichlorosilane, dodecyltrichlorosilane, ethyltrichlorosilane, n-octyltrichlorosilane, phenyltrichlorosilane, trichloro-2-cyanoethylsilane, trichlorohexylsilane, trichloro(methyl)silane, trichlorooctadecylsilane, trichloro(propyl)silane, and trichlorotetradecylsilane.

For example, commercially available compounds manufactured by Tokyo Chemical Industry Co., Ltd. can be used as the above-described functional silane. The functional silanes can be used alone or in combination of two or more.

Next, a cell surface binding substance is bound to a nanoparticle made of the complex of polysiloxane and porphyrin as needed. Specifically, an aqueous solution of the cell surface binding substance is added to an aqueous solution of the nanoparticles made of the complex of polysiloxane and porphyrin, and the resultant mixture is stirred at a temperature of 4 to 50°C for 1 to 24 hours and reacted. For example, folic acid serving as the cell surface binding substance may be bound to the nanoparticle made of the complex of polysiloxane and porphyrin. A cell surface binding substance modified with a functional group capable of binding to the functional group present on the surface of the nanoparticle made of the complex of polysiloxane and porphyrin is used. For example, when the functional silane used to form the complex of polysiloxane and porphyrin is a silane having a thiol group such as 3-mercaptopropyl(dimethoxy)methylsilane, (3-mercaptopropyl)triethoxysilane, or (3-mercaptopropyl)trimethoxysilane, a compound obtained by respectively binding a maleimide group and folic acid to both of the termini of polyethylene glycol (PEG) can be used as the folic acid.

In the above-mentioned reaction, the molar ratio of the functional group of the nanoparticle made of the complex of polysiloxane and porphyrin that is covalently bound to folic acid to the functional group with which the folic acid is modified and that is covalently bound to the complex of polysiloxane and porphyrin (the functional group of the nanoparticle made of the complex of polysiloxane and porphyrin that is covalently bound to folic acid:the functional group with which the folic acid is modified) is preferably 1:1 to 3:1, more preferably 1:1 to 2:1, and even more preferably 1:1. It should be noted that when the fluorescent probe for flow cytometry of the present invention includes the fluorescent dye b in addition to porphyrin, the fluorescent dye b may be bound to the complex of polysiloxane and porphyrin together with the folic acid.

When TCPP is used as the porphyrin, it is preferable that the carboxyl group of TCPP forms an amide bond in the complex of polysiloxane and porphyrin, and it is more preferable that TCPP is incorporated into a siloxane network (main chain of polysiloxane) by an amide bond. In the present invention, the structure of the fluorescent probe such as the complex of polysiloxane and porphyrin can be analyzed using Fourier transform infrared spectrophotometry as described later.

The fluorescent probe for flow cytometry preferably includes four pyrrole ring structure moieties (porphine) in porphyrin in an amount of 1 mass% or more, and more preferably 5 to 90 mass%, from the viewpoint of suitability for flow cytometry analysis using a flow cytometer, but there is no particular limitation thereto. As described later, in the present invention, analyzing a fluorescent probe such as the nanoparticle made of siloxane and porphyrin through thermogravimetric-differential thermal analysis makes it possible to measure the content of a fluorescent dye such as porphyrin and a carrier molecule such as silica polymer.

The fluorescent probe for flow cytometry preferably has an average particle diameter of 3 to 250 nm, and more preferably 10 to 80 nm, from the viewpoint of the cells being easy to label, but there is no particular limitation thereto. In the present invention, the average particle diameter is measured through dynamic light scattering.

### Method for screening fluorescence-labeled cells

A method for screening fluorescence-labeled cells of the present invention includes a step of fluorescently labeling cells with the above-mentioned fluorescent probe for flow cytometry, and a step of screening the fluorescence-labeled cells labeled with the fluorescent probe for flow cytometry using a flow cytometer.

First, cells are fluorescently labeled with the fluorescent probe. Cells can be labeled by binding the fluorescent probe for flow cytometry to the cell surface or causing cells to take up the fluorescent probe for flow cytometry. When the fluorescent probe is bound to the cell surface, the fluorescent probe includes a cell surface binding substance. For example, when the fluorescent probe includes folic acid, cancer cells such as HeLa S3 cells derived from human cervical cancer and HCT116 cells derived from human large intestine cancer can be labeled by binding the fluorescent probe to the surfaces of these cancer cells. Specifically, culturing cells in a cell culture medium containing the fluorescent probe makes it possible to label the cells with the fluorescent probe. Examples of the cells include stem cells such as iPS cells and ES cells, and disease-related cells such as cancer cells and cirrhotic cells.

Next, the fluorescence-labeled cells labeled with the fluorescent probe for flow cytometry are screened using a flow cytometer. Screening of the fluorescence-labeled cells using a flow cytometer is performed as follows: cells that have been subjected to a fluorescence labeling operation are irradiated with an excitation light with an excitation wavelength of 350 to 650 nm and then fluorescence is detected. Specifically, cells that have been subjected to a fluorescence labeling operation using a fluorescent probe for flow cytometry are supplied to a flow cytometer and analyzed through flow cytometry analysis using a predetermined excitation wavelength and a predetermined fluorescence wavelength, such as an excitation wavelength of 350 to 650 nm and a fluorescence wavelength of 400 to 800 nm, thus making it possible to screen the fluorescence-labeled cells that have been fluorescently labeled with the fluorescent probe for flow cytometry. There is no particular limitation on the flow cytometer, and any flow cytometer can be used.

### Examples

Hereinafter, the present invention will be specifically described by way of examples. It should be noted that the present invention is not limited to the following examples.

### Reagents

All of tetrakis(4-carboxyphenyl)porphyrin (TCPP), (3-mercaptopropyl)trimethoxysilane (MPTMS), N,N-dimethylformamide (DMF), polyethylene glycol (PEG) in which folic acid and a maleimide group are respectively bound to both of the termini (molecular weight: 3400; also referred to simply as "FA-PEG-Mal" hereinafter), and indocyanine green to which a maleimide group is bound (molecular weight: 853; also referred to simply as "ICG-Mal" hereinafter) were obtained from Tokyo Chemical Industry Co., Ltd. The following are the structural formulae of MPTMS, FA-PEG-Mal, and ICG-Mal.

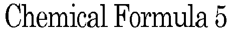

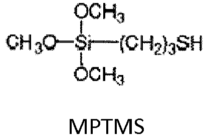

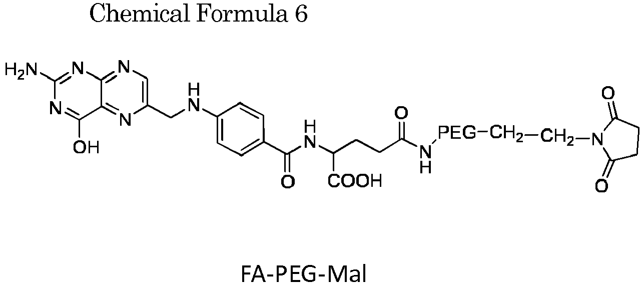

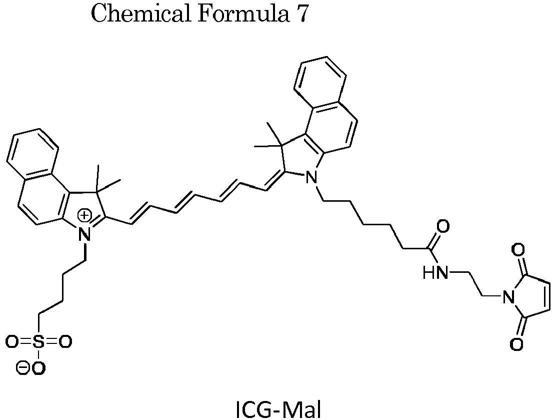

### Example 1

### Production of fluorescent probe

### Production of complex of carrier molecule and porphyrin

(1) APTES (462 µmol) was added to a DMF solution of TCPP (3.8 mM: 32 mL) obtained by dissolving TCPP in DMF, and then N,N'-dipropylcarbodiimide (480 µmol) and N-hydroxysuccinimide (480 µmol) were added thereto. The resultant mixture was stirred at 50°C for 24 hours.
(2) Next, 200 µL (porphyrin-silane: 0.75 µmol) of the DMF solution of porphyrin-silane obtained as described above was mixed with 5 µl (0.027 mmol) of MPTMS. To the obtained mixed solution, 500 µL of DMF and 300 µL of an aqueous solution of ammonia (concentration: 28 mass%, pH 11) were added, and a reaction was carried out at 80°C for 24 hours.
(3) After 24 hours, the product was collected as a precipitate through centrifugation (15000 rpm for 20 minutes). Then, the product was washed with water and ethanol several times, and was finally dispersed in 1 mL of water.
(4) The obtained substance was a complex of polysiloxane and porphyrin in which the polysiloxane serves as a carrier molecule and the porphyrin serves as a fluorescent dye, and the porphyrin is covalently bound to the polysiloxane. From the results of the measurement performed through dynamic light scattering ("DelsaMax Pro" manufactured by Beckman Coulter), it was found that the obtained substance was a fluorescent hybrid nanoparticle (also referred to simply as "PPS HNPs" hereinafter) with an average particle diameter of about 40 nm.

Step of binding fluorescent dye b to complex of carrier molecule and porphyrin
(1) To 1 mL of the aqueous dispersion of the PPS HNPs obtained as described above, 63 µl of an aqueous solution of ICG-Mal (concentration: 2 mg/mL, ICG-Mal: 1.48×10⁻⁴ mmol) was added. The resultant mixture was stirred at 30°C for 3 hours to carry out a reaction.
(2) After the reaction, the product was collected as a precipitate through centrifugation (15000 rpm for 20 minutes). Then, the product was washed with water several times, and was finally dispersed in 1 mL of water.
(3) The obtained substance was a complex (fluorescent probe) in which the porphyrin and the indocyanine green (fluorescent dye b) were covalently bound to the polysiloxane. From the results of the measurement performed through dynamic light scattering ("DelsaMax Pro" manufactured by Beckman Coulter), it was found that the obtained substance was a fluorescent hybrid nanoparticle (also referred to simply as "ICG-PPS HNPs" hereinafter) with an average particle diameter of about 50 nm.

### Example 2

### Production of fluorescent probe

### Production of complex of carrier molecule and porphyrin

PPS HNPs was obtained in the same manner as in Example 1.

Step of binding fluorescent dye b and cell surface binding substance to complex of carrier molecule and porphyrin
(1) To 1 mL of the aqueous dispersion of the PPS HNPs obtained as described above, 251 µl of an aqueous solution of FA-PEG-Mal (concentration: 2 mg/mL, FA-PEG-Mal: 1.48×10⁻⁴ mmol) and 63 µl of an aqueous solution of ICG-Mal (concentration: 2 mg/mL, ICG-Mal: 1.48×10⁻⁴ mmol) were added. The resultant mixture was stirred at 30°C for 3 hours.
(2) After the reaction, the product was collected as a precipitate through centrifugation (15000 rpm for 20 minutes). Then, the product was washed with water several times, and was finally dispersed in 1 mL of water.
(3) The obtained substance was a complex (fluorescent probe) in which the porphyrin, the indocyanin green (fluorescent dye b), and the folic acid (cell surface binding substance) are covalently bound to the polysiloxane. From the results of the measurement performed through dynamic light scattering ("DelsaMax Pro" manufactured by Beckman Coulter), it was found that the obtained substance was a fluorescent hybrid nanoparticle (also referred to simply as "FA-PEG/ICG-PPS HNPs" hereinafter) with an average particle diameter of about 65 nm.

### Confirmation through Fourier transform infrared spectrophotometry

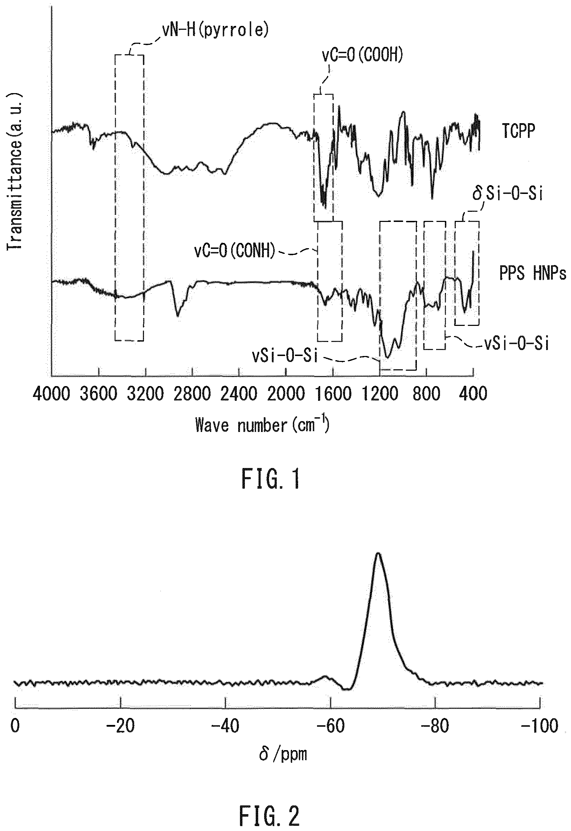

The PPS HNPs obtained in Examples 1 and 2 and the TCPP used as a raw material were analyzed using a Fourier transform infrared spectrophotometer ("NEXUS470" manufactured by Nicolet). FIG. 1 shows FT-IR spectra of the PPS HNPs and the TCPP. It was found from the FT-IR spectra shown in FIG. 1 that both of them exhibited a peak originating from a pyrrole ring at 3430 to 3250 cm⁻¹. Since a peak originating from carboxylic acid (carboxyl group) of the TCPP at 1800 to 1640 cm⁻¹ disappeared and a peak originating from amide at 1740 to 1620 cm⁻¹ appeared in the FT-IR spectrum of the PPS HNPs, it was confirmed that the complex of polysiloxane and porphyrin in which a carboxyl group of the TCPP forms an amide bond and is thus covalently bound to the siloxane (that is, the complex of polysiloxane and porphyrin formed through hydrolysis and condensation of the porphyrin-silane and the MPTMS) was reliably synthesized. In addition, peaks originated from siloxane bonds appeared at 1260 to 1010 cm⁻¹ (vSi-O-Si), 870 to 740 cm⁻¹ (vSi-O-Si), and 540 to 410 cm⁻¹ (σSi-O-Si) in the FT-IR spectrum of the PPS HNPs. That is, it was confirmed that the porphyrin-silane and the MPTMS (silane having a functional group) formed a siloxane network in PPS HNPs, and the porphyrin was incorporated into the siloxane network by an amide bond.

The structure of the PPS HNPs obtained in Examples 1 and 2 was analyzed through ²⁹Si solid-state NMR. FIG. 2 shows a ²⁹Si solid-state NMR spectrum of the PPS HNPs. It was confirmed from FIG. 2 that peaks appeared at -58 ppm and -69 ppm. It was inferred that the peak at -58 ppm originated from the T² structure represented by a chemical formula below, and the peak at -69 ppm originated from the T³ structure represented by a chemical formula below. It was confirmed from this result that a portion of the PPS HNPs was constituted by Si with the T₂ structure, but most of the MPTMS and porphyrin-silane were subjected to hydrolysis and polycondensation. That is, the porphyrin was covalently bound to the siloxane, and the complex was thus formed.

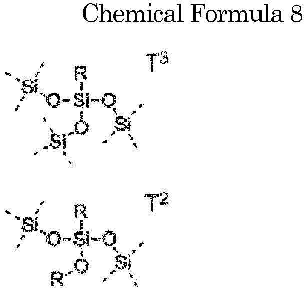

### Thermogravimetric-differential thermal analysis

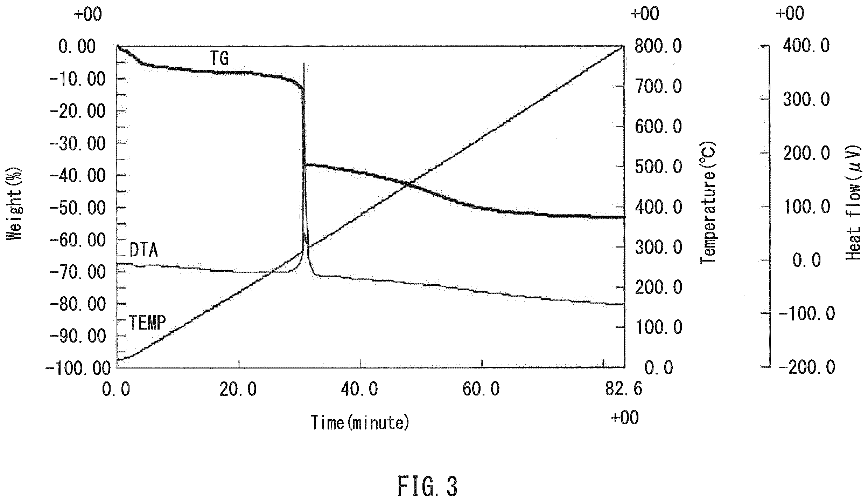

The PPS HNPs was analyzed through thermogravimetric-differential thermal analysis (TG-DTA) using a thermal analyzer (TG8120, manufactured by Rigaku Corporation). FIG. 3 shows a TG (thermogravimetric) curve and a DTA (differential thermal analysis) curve of the PPS HNPs. It is inferred from the analysis results that the weight reduction at 100°C was caused due to adsorbed water evaporating, the weight reduction at 300°C was due to the combustion of the aminopropyl moiety and the mercaptopropyl moiety, the weight reduction at 300 to 400°C was due to the decomposition of the pyrrole ring structure moieties (porphine) in the porphyrin, and the remaining portion was constituted by the siloxane moiety. Specifically, it was inferred that the PPS HNPs contained the adsorbed water in an amount of 7 wt%, the aminopropyl moiety and mercaptopropyl moiety in an amount of 29 wt%, the pyrrole ring structure moieties (porphine) in the porphyrin in an amount of 17 wt%, and the polysiloxane moiety in an amount of 47 wt%.

### Quantification of FA-PEG and ICG bound to PPS HNPs

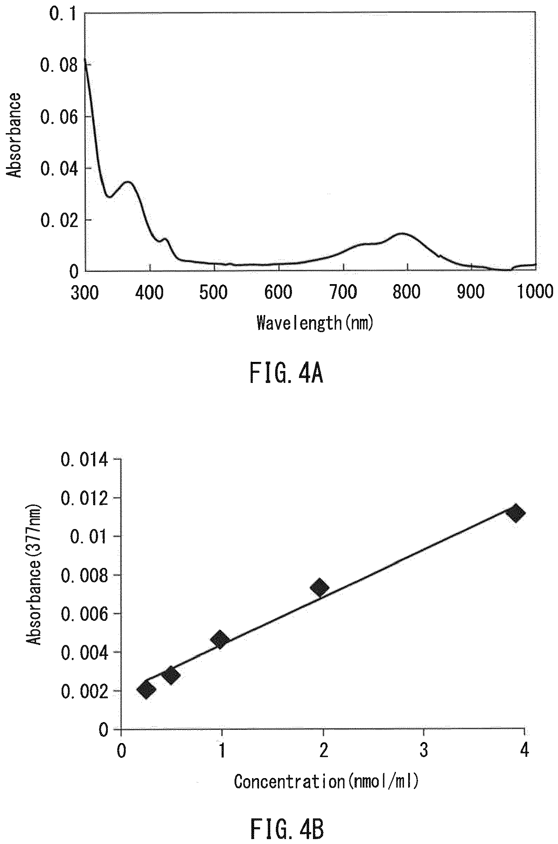

In order to determine the amounts of the FA-PEG and the ICG bound to the PPS HNPs, an absorption spectrum of the supernatant (1 mL) after the reaction was measured using a spectrophotometer ("V-570" manufactured by JASCO Corporation), and the amounts of unreacted FA-PEG-Mal and ICG-Mal were calculated. FIG. 4A shows the absorption spectrum of the supernatant. Regarding the FA-PEG-Mal, the extinction coefficient of folic acid in an aqueous solution was unknown, and therefore, a calibration curve of folic acid at a peak wavelength of 377 nm was produced, and the concentration was calculated using this calibration curve. FIG. 4B shows the calibration curve for folic acid. In FIG. 4A, the absorbance at 377 nm is 0.032, and it was thus determined that the concentration of the folic acid in the supernatant was 12 nmol/mL. The amount of the FA-PEG-Mal used was 148 nmol, and therefore, it was confirmed that 92% of the FA-PEG-Mal was reacted. Next, regarding the ICG-Mal, the concentration of the ICG was calculated based on the formula of Lambert-Beer law, namely A₈₀₀=ε₈₀₀cL using the molar extinction coefficient of the ICG in an aqueous solution at 800 nm, namely ε₈₀₀=147000 L/mol·cm. As a result, the concentration of the ICG was 0.094 nmol/mL. The amount of the ICG-Mal used was 148 nmol, and therefore, it could be estimated that 99.9% of the ICG-Mal was reacted. It was determined from these calculation results that 92% of the FA-PEG and substantially 100% of the ICG were bound to the PPS HNPs. When the amount of the ICG bound to the PPS HNPs was determined in Example 1 in the same manner, it was confirmed that substantially 100% of the ICG was bound to the PPS HNPs.

### Confirmation of absorption spectrum through spectroscopy

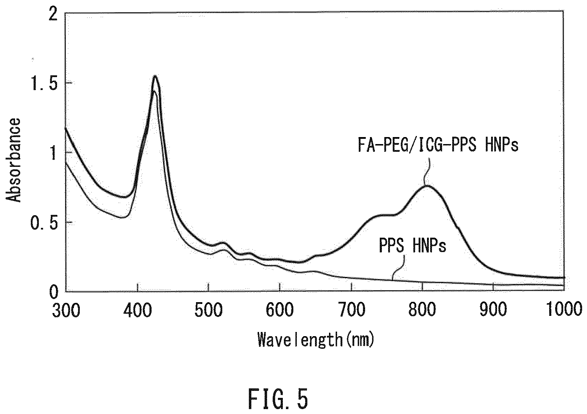

The absorption spectra of the aqueous dispersion (also referred to as "aqueous solution") of the PPS HNPs obtained in Examples 1 and 2 and the aqueous solution of the FA-PEG/ICG-PPS HNPs obtained in Example 2 were measured using a spectrophotometer ("V-570" manufactured by JASCO Corporation). FIG. 5 below shows the results. It was confirmed from the absorption spectra of the PPS HNPs and the FA-PEG/ICG-PPS HNPs shown in FIG. 5 that a peak originating from ICG appeared around a wavelength of 700 to 900 nm due to the ICG being bound to the PPS HNPs. Also, it was confirmed that a peak around 380 to 650 nm originating from porphyrin barely changed in the FA-PEG/ICG-PPS HNPs.

### Confirmation of wavelength characteristics of fluorescent probe

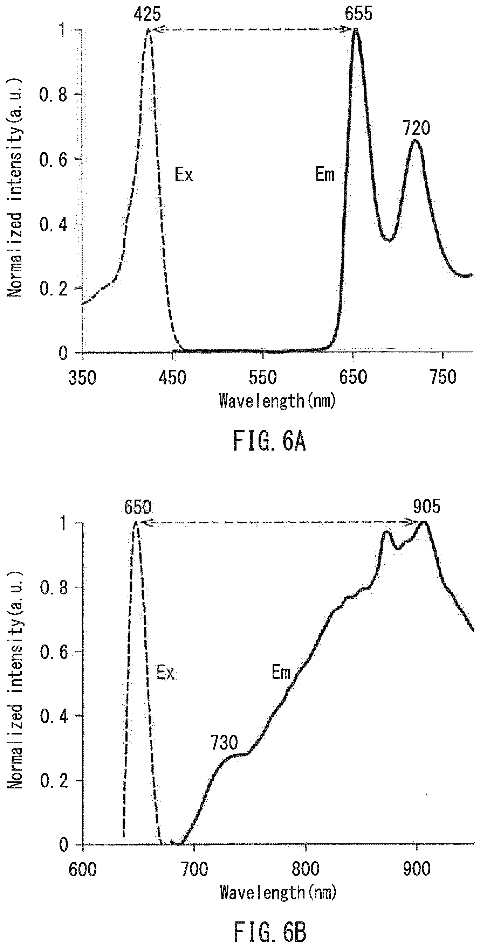

The aqueous solution of the FA-PEG/ICG-PPS HNPs obtained in Example 2 was used to analyze the excitation wavelength spectrum and the fluorescence wavelength spectrum of the FA-PEG/ICG-PPS HNPs using a fluorescence spectrophotometer FP-6600 manufactured by JASCO Corporation. FIG. 6 shows the results. FIG. 6A shows the wavelength characteristics on a short wavelength side to be used in in vitro fluorescence imaging, and FIG. 6B shows the wavelength characteristics on a long wavelength side to be used in in vivo fluorescence imaging. It was found from FIGS. 6A and 6B that Stokes shift was large on both the short wavelength side with a maximum excitation wavelength of 425 nm and a maximum fluorescence wavelength of 655 nm and the long wavelength side with a maximum excitation wavelength of 650 nm and a maximum fluorescence wavelength of 905 nm. It was confirmed that both the ICG-PPS HNPs and the FA-PEG/ICG-PPS HNPs were fluorescent probes that include porphyrin and ICG (fluorescent dye b) bound to a carrier molecule (polysiloxane) and in which fluorescence resonance energy transfer does not occur between the two fluorescent dyes.

### Analysis using fluorescence microscope

### (1) Cells

RAW264.7 cells derived from a mouse macrophage, which are homologous to mouse cells, and HeLa S3 cells derived from human cervical cancer and HCT116 cells derived from human large intestine cancer, which are not homologous to mouse cells, were used. In the examples, cells and cell culture media were obtained from Sigma-Aldrich. Cell culture dishes manufactured by Thermo Fisher Scientific were used.

### (2) Fluorescent probe labeling of cells

(a) Cells (0.3×10⁵ cells/mL) were seeded into a cell culture medium (DMEM medium containing 10% FBS) in a cell culture dish (with a diameter of 35 mm) and cultured overnight.
(b) The cultured cells were transferred into a cell culture medium (DMEM medium containing 10% FBS) containing the fluorescent probe (50 µg/mL), and cultured for 24 hours. At this time, the RAW264.7 cells were transferred into a cell culture medium containing the fluorescent probe ICG-PPS HNPs, and the HeLa S3 cells and the HCT116 cells were transferred into a cell culture medium containing the fluorescent probe FA-PEG/ICG-PPS HNPs.
(c) The supernatant was removed, and the cultured cells were washed with 2 mL of phosphate buffered saline (1×PBS, pH 7.4: "D-PBS(-) (045-29795)" manufactured by Wako Pure Chemical Corporation).
(d) The supernatant was removed, and 2 mL of 4% paraformaldehyde (PFA) was added to the cultured cells. The cells were left to stand at room temperature (20±5°C) for 30 minutes.
(e) The supernatant was removed, and 1 mL of 1×PBS was added to the cultured cells. This step was repeated three times.

### (3) Observation using fluorescence microscope

The fluorescence-labeled cells labeled with the fluorescent probe were observed using a fluorescence microscope EVOS (registered trademark) FL Cell Imaging System manufactured by Life Technologies. All the cells were observed using an excitation wavelength of 422 nm and a fluorescence wavelength of 655 nm. FIG. 7 shows the results.

FIG. 7A shows a bright field image (Bright field) of RAW264.7 cells, a fluorescence image (DAPI) of cell nuclei using a fluorescent dye DAPI (4,6-diamidino-2-phenylindole), fluorescence imaging using the ICG-PPS HNPs (fluorescent probe), and an image (Merge) obtained by merging these three images together. It was found from the bright field image shown in FIG. 7A that the cells were present. In the DAPI image, the cell nuclei stained with DAPI were confirmed. It was found from the ICG-PPS HNPs image that the cells were labeled with the fluorescent probe. It was found from the Merge image that fluorescence of the fluorescent probe was observed more frequently in the cytoplasm in the RAW264.7 cells, and the nuclei did not emit fluorescence. It was confirmed from these results that the ICG-PPS HNPs (fluorescent probe) was taken up by the cell and stayed in the cytoplasm. The RAW264.7 cells are macrophage cells, and therefore, it was thought that the fluorescent probe was taken up by the cell due to its phagocytic activity, and fluorescent probes accumulated in the cytoplasm.

FIG. 7B shows a bright field image (Bright field) of HeLa S3 cells, a fluorescence image (DAPI) of cell nuclei using a fluorescent dye DAPI, fluorescence imaging using the FA-PEG/ICG-PPS HNPs fluorescent probe, and an image (Merge) obtained by merging these three images together. It was found from the bright field image shown in FIG. 7B that the cells were present. In the DAPI image, cell nuclei stained with DAPI were confirmed. It was found from the FA-PEG/ICG-PPS HNPs fluorescent probe image that the cells were labeled with the fluorescent probe. It was found from the Merge image that the fluorescent probe emitted fluorescence from the entire HeLa S3 cell including the cell nucleus. It was revealed from these results that the fluorescent probe was bound to the cell surface, and light emission from the entire cell was thus observed. It was thought that cancer cells have a receptor to which folic acid binds on the cell surface, and the fluorescent probe (FA-PEG/ICG-PPS HNPs) was bound to the surface of the HeLa S3 cell, which is a cancer cell, via folic acid.

FIG. 7C shows a bright field image (Bright field) of HCT116 cells, a fluorescence image (DAPI) of cell nuclei using a fluorescent dye DAPI, fluorescence imaging using the FA-PEG/ICG-PPS HNPs fluorescent probe, and an image (Merge) obtained by merging these three images together. It was found from the bright field image shown in FIG. 7C that the cells were present. In the DAPI image, cell nuclei stained with DAPI were confirmed. It was found from the FA-PEG/ICG-PPS HNPs fluorescent probe image that the cells were labeled with the fluorescent probe. It was found from the Merge image that the fluorescent probe emitted fluorescence from the entire HCT116 cell including the cell nucleus. It was revealed from these results that the fluorescent probe was bound to the cell surface, and light emission from the entire cell was thus observed. It was thought that cancer cells have a receptor to which folic acid binds on the cell surface, and the fluorescent probe (FA-PEG/ICG-PPS HNPs) was bound to the surface of the HCT116 cell, which is a cancer cell, via folic acid.

### Analysis using flow cytometer

### (1) Cells

RAW264.7 cells derived from a mouse macrophage, which are homologous to mouse cells, and HeLa S3 cells derived from human cervical cancer and HCT116 cells derived from human large intestine cancer, which are not homologous to mouse cells, were used.

### (2) Fluorescent probe labeling of cells

(a) Cells were seeded into a cell culture medium (DMEM medium containing 10% FBS) in a cell culture dish (with a diameter of 35 mm) in a seeding amount shown in Table 1 below and cultured overnight.
(b) The cultured cells were transferred into a cell culture medium (DMEM medium containing 10% FBS) containing the fluorescent probe (50 µg/mL) shown in Table 1 below, and cultured for another 48 hours.
(c) The cells were collected from the cell culture dish. At this time, the HeLa S3 cells and the HCT116 cells were collected using a 0.25% trypsin/EDTA solution.
(d) After the collected solution was centrifuged at 1000 revolutions for 5 minutes, the supernatant was removed, and the cells were suspended in 400 µL of a cell culture medium (DMEM medium containing 10% FBS).
(e) After the suspension was centrifuged at 1000 revolutions for 5 minutes, the supernatant was removed, and 1 mL of 4% PFA was added to the cells. The cells were left to stand at room temperature (20±5°C) for 30 minutes.
(f) After the resuspension was centrifuged at 1000 revolutions for 5 minutes, the supernatant was removed, and 1 mL of 1×PBS was added to the cells. This step was repeated three times.

**Table 1**

| Type of cells | Origin | Fluorescent probe | Localization of fluorescent probe | Cell seeding amount (cells/mL) |
|---|---|---|---|---|
| RAW264.7 cells | Mouse | ICG-PPS HNPs | Intracellular | 2×10⁵ |
| HeLa S3 cells | Human | FA-PEG/ICG-PPS HNPs | On cell surface | 2×10⁵ |
| HCT116 cells | Human | FA-PEG/ICG-PPS HNPs | On cell surface | 5×10⁵ |

### Observation using flow cytometer

The cells labeled with the fluorescent probe were observed using a flow cytometer LSR Fortessa X-20 manufactured by BD.

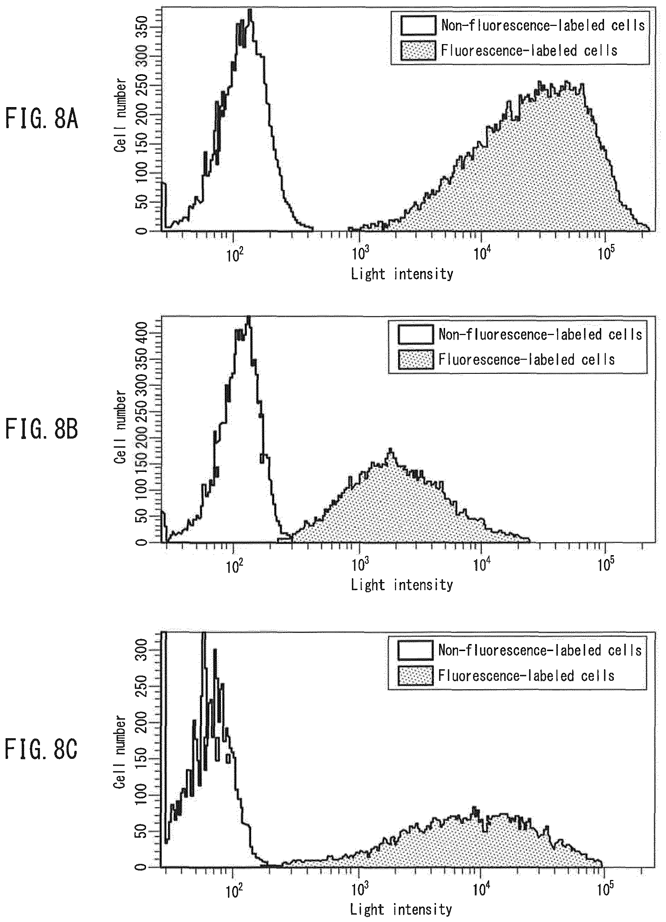

FIG. 8 shows the results of the observation using an excitation wavelength of 405 nm and a fluorescence wavelength of 670 nm. FIG. 8A shows the result from the RAW264.7 cells labeled with the ICG-PPS HNPs, FIG. 8B shows the result from the HeLa S3 cells labeled with the FA-PEG/ICG-PPS HNPs, and FIG. 8C shows the result from the HCT116 cells labeled with the FA-PEG/ICG-PPS HNPs.

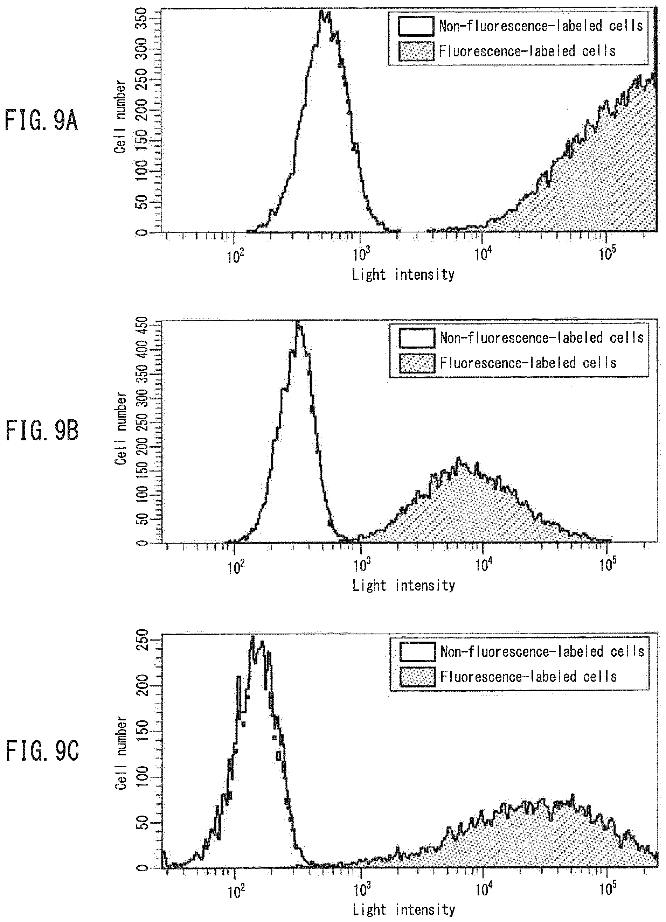

FIG. 9 shows the results of the observation using an excitation wavelength of 405 nm and a fluorescence wavelength of 710 nm. FIG. 9A shows the result from the RAW264.7 cells labeled with the ICG-PPS HNPs, FIG. 9B shows the result from the HeLa S3 cells labeled with the FA-PEG/ICG-PPS HNPs, and FIG. 9C shows the result from the HCT116 cells labeled with the FA-PEG/ICG-PPS HNPs.

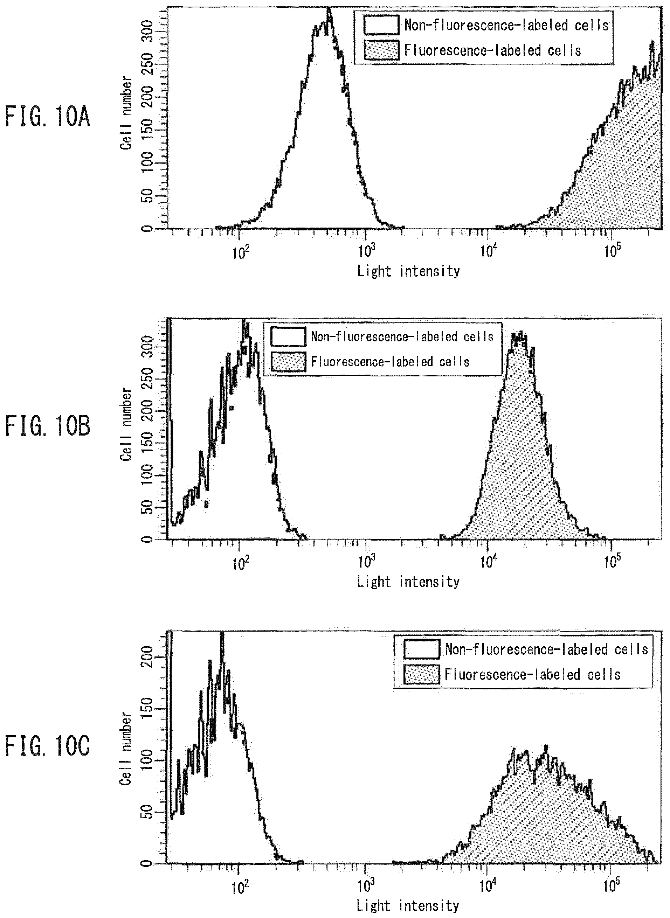

FIG. 10 shows the results of the observation using an excitation wavelength of 405 nm and a fluorescence wavelength of 780 nm. FIG. 10A shows the result from the RAW264.7 cells labeled with the ICG-PPS HNPs, FIG. 10B shows the result from the HeLa S3 cells labeled with the FA-PEG/ICG-PPS HNPs, and FIG. 10C shows the result from the HCT116 cells labeled with the FA-PEG/ICG-PPS HNPs.

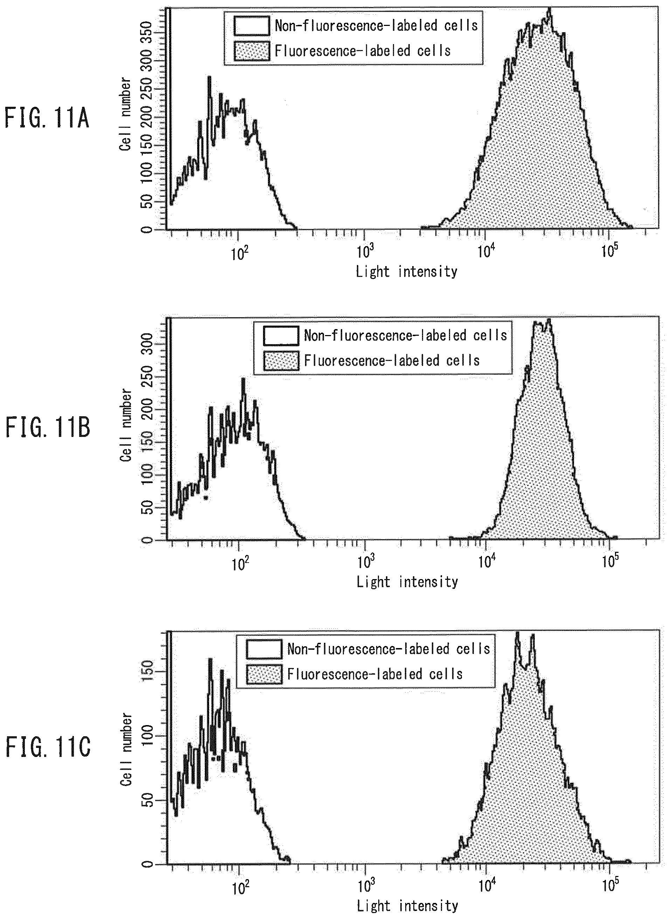

FIG. 11 shows the results of the observation using an excitation wavelength of 640 nm and a fluorescence wavelength of 780 nm. FIG. 11A shows the result from the RAW264.7 cells labeled with the ICG-PPS HNPs, FIG. 11B shows the result from the HeLa S3 cells labeled with the FA-PEG/ICG-PPS HNPs, and FIG. 11C shows the result from the HCT116 cells labeled with the FA-PEG/ICG-PPS HNPs.

As is clear from FIGS. 8 to 11, in all of the cell samples, the fluorescence-labeled cells and the non-fluorescence-labeled cells were separately observed using any excitation wavelength and any fluorescence wavelength. Light intensities were different by a factor of about 100 to 10000 times. After the cells were labeled with the fluorescent probe, the fluorescence-labeled cells and the non-fluorescence-labeled cells could be clearly distinguished and separated. Using a flow cytometer makes it possible to screen only the fluorescence-labeled cells labeled with the fluorescent probe.

## Claims

1. A fluorescent probe for flow cytometry, comprising:
a carrier molecule; and
porphyrin bound to the carrier molecule,
wherein an excitation wavelength of the fluorescent probe for flow cytometry is in a range of 350 to 650 nm.

2. The fluorescent probe for flow cytometry according to claim 1, wherein the carrier molecule is polysiloxane.

3. The fluorescent probe for flow cytometry according to claim 1 or 2, which is specifically bound to a surface of a cell so that the cell is labeled.

4. The fluorescent probe for flow cytometry according to claim 1 or 2, which is taken up by a cell so that the cell is labeled.

5. The fluorescent probe for flow cytometry according to any one of claims 1 to 4, wherein a fluorescence wavelength is in a range of 400 to 800 nm.

6. A method for screening fluorescence-labeled cells using a flow cytometer, comprising:
a step of fluorescently labeling cells with a fluorescent probe for flow cytometry; and
a step of screening fluorescence-labeled cells labeled with the fluorescent probe for flow cytometry using a flow cytometer,
wherein the fluorescent probe for flow cytometry comprises a carrier molecule and porphyrin bound to the carrier molecule,
an excitation wavelength of the fluorescent probe for flow cytometry is in a range of 350 to 650 nm, and
the screening of the fluorescence-labeled cells using a flow cytometer is performed by irradiating the fluorescence-labeled cells with an excitation light with a wavelength of 350 to 650 nm and detecting fluorescence.

7. The method for screening fluorescence-labeled cells according to claim 6, wherein the carrier molecule is polysiloxane.

8. The method for screening fluorescence-labeled cells according to claim 6 or 7, wherein the fluorescent probe for flow cytometry is specifically bound to a surface of a cell so that the cell is labeled.

9. The method for screening fluorescence-labeled cells according to claim 6 or 7, wherein the fluorescent probe for flow cytometry is taken up by a cell so that the cell is labeled.

10. The method for screening fluorescence-labeled cells according to any one of claims 6 to 9, wherein a fluorescence wavelength of the fluorescent probe for flow cytometry is in a range of 400 to 800 nm.
